# EUROPEAN PATENT APPLICATION

(11) **EP 1 769 971 A1**
(43) Date of publication of application: **04.04.2007**
(21) Application number: 05753458.8
(22) Date of filing: 22.06.2005
(51) Int. Cl.: B60R 11/02, B60R 16/02

(54) **ON-VEHICLE IMAGE DISPLAY UNIT**

(30) Priority: 23.06.2004 JP 2004185065
(71) Applicant: MATSUSHITA ELECTRIC INDUSTRIAL CO., LTD., Osaka 571-8501 (JP)
(72) Inventor: MORIMOTO, Akihiro c/o Matsuhita El. Ind. Co., Ltd., Chuo-ku, Osaka-shi, Osaka 540-6319 (JP); IBARAKI, Susumu c/o Matsuhita El. Ind. Co., Ltd., Chuo-ku, Osaka-shi, Osaka 540-6319 (JP); HAMADA, Hiroyuki c/o Matsuhita El. Ind. Co., Ltd., Chuo-ku, Osaka-shi, Osaka 540-6319 (JP); OTSUKA, Masakazu c/o Matsuhita El. Ind. Co., Ltd., Chuo-ku, Osaka-shi, Osaka 540-6319 (JP); KUWABARA, Takashi c/o Matsuhita El. Ind. Co., Ltd., Chuo-ku, Osaka-shi, Osaka 540-6319 (JP); NAKAGAWA, Seiichi c/o Matsuhita El. Ind. Co., Ltd., Chuo-ku, Osaka-shi, Osaka 540-6319 (JP); NAGASHIMA, Kazumasa Matsuhita El. Ind. Co., Ltd., Chuo-ku, Osaka-shi, Osaka 540-6319 (JP)
(74) Representative: Pautex Schneider, Nicole Véronique
(86) International application number: PCT/JP2005/011417
(87) International publication number: WO 2006/001295

(57) **Abstract**

It is an object of the present invention to provide an image display apparatus which can reduce a burden to be imposed on vehicle occupants by informing the vehicle occupants about current and next travel states of a vehicle while allowing the vehicle occupants to watch TV or the like in the vehicle. In the on-vehicle image display apparatus, the image controlling means 13 controls the on-screen position of the image produced by the image producing means 11 on the basis of the judgments made by the travel state detecting means 12 on whether or not the vehicle is turning to the right, or about to turn to the right, whether or not the vehicle is turning to the left, or about to turn to the left, whether or not the vehicle is being accelerated, or about to be accelerated, and whether or not the vehicle is being decelerated, or about to be decelerated. As an example, the image controlling means 13 shifts, in a leftward direction, the on-screen position of the vehicle if the judgment is made that the vehicle is turning to the left, and to shift, in a rightward direction, the on-screen position of the vehicle if the judgment is made that the vehicle is turning to the right.

## Description

### TECHNICAL FIELD OF THE INVENTION

The present invention relates to an on-vehicle image display apparatus to be mounted on a vehicle, and for producing an image to be watched by vehicle occupants in the vehicle.

### DESCRIPTION OF THE RELATED ART

In recent years, there have been increasing vehicles respectively equipped with display units each of which displays various information on a screen, and more specifically the vehicles respectively equipped with navigation apparatuses each of which has a display unit for displaying a road map on a screen while allowing the center of the screen to correspond to its current position, or the vehicles respectively equipped with display units each of which is provided for a front passenger or rear passengers, and each of which displays an image as a television, a video tape recorder, a DVD recorder, a game machine and the like.

In moving vehicle, vehicle occupants are being swung in varying degrees by a vibratory motion resulting from an engine and other driving mechanism, or resulting from the fact that its chassis is rocked, shaken, or shocked by inequalities of the ground, a surface profile of the road, curbstones and the like, and additionally resulting from an acceleration and a deceleration.

In the above-mentioned surroundings, a central nerve system of each vehicle occupant tends to be messed up (sensory confusion), without sensing his or her position and movement, by recognizing that current sensory information is different from sensory information previously-accumulated in his or her central nerve, and then tries to adapt to his or her environmental change by acknowledging the current sensory information. It is generally believed that he or she gets motion sickness through this process. If, for example, one vehicle occupant keeps his or her eyes trained on a book, and reads the book in moving vehicle, he or she is liable to get motion sickness by reason that visual information is different from vestibular information or somatic sensory information. As one method of preventing from the sensory confusion in moving vehicle, it is generally believed that each vehicle occupant can close his or her eyes, or stare into the distance as much as possible. Additionally, it is generally believed that the driver is hard to get motion sickness in comparison with passengers by reason that the driver feels tense by driving a vehicle, and tries to reduce a change of an acceleration by estimating a motion of the vehicle, and by changing his or her head position on the basis of its estimation (See non-patent document 1).

As a method helpful in preventing motion sickness, there have been known a wide variety of anti-motion sickness drugs, one typical example of which is known as an antihistamine, and disclosed in, for example, non-patent document 2.

As an apparatus helpful in preventing motion sickness, there has been known a wide variety of headrests, one typical example of which is disclosed in, for example, patent document 1. The headrest is movable with respect to a seat in response to a travel state of a vehicle while the vehicle goes around a curve.

As another apparatus helpful in preventing motion sickness, there have been known a wide variety of display apparatuses, one typical example of which is disclosed in patent document 2, and informs vehicle occupants other than a driver about current and next travel states of a vehicle by indicating information on whether or not the vehicle is turning to the right or the left, or about to turn to the right or the left, whether or not the vehicle is being accelerated or decelerated, or about to be accelerated or decelerated, and whether or not the vehicle is stopped.
patent document 1: Jpn. unexamined patent publication No. H07-252 (FIG. 1)
patent document 2: Jpn. unexamined patent publication No. 2002-154350 (FIG. 1)
non-patent document 1: "motion sickness and space motion sickness" written by Ryo Matsunaga, and Noriaki Takeda, otorhinolaryngological clinic, Vol. 81, No. 8, pages 1095-1120, 1998
non-patent document 2: Toyokiti Kinniti "internal therapy" JOHNS, Vol. 6, No. 9, 1990-9

### DISCLOSURE OF THE INVENTION

### PROBLEMS TO BE SOLVED BY THE INVENTION

The above-mentioned anti-motion sickness drug, however, encounters such a problem that the driver tends to feel sleepy as a side effect resulting from this drug.

The above-mentioned headrest, however, encounters such a problem that the vehicle occupants cannot escape from being rocked in moving vehicle.

The above-mentioned apparatus, however, encounters such a problem that the vehicle occupants cannot pay attention to the indicated information while watching TV or the like in a moving vehicle. As a result, the vehicle occupants tend to have motion sickness.

It is, therefore, an object of the present invention to provide an image display apparatus which can inform and remind the vehicle occupants about the current and next travel states of the vehicle while allowing the vehicle occupants to watch TV or the like in a moving vehicle, and reduce a burden to be imposed on the vehicle occupants.

### MEANS FOR SOLVING THE PROBLEMS

The on-vehicle image display apparatus according to the present invention, comprises, as an apparatus mounted on a vehicle, image producing means for producing an image, travel state detecting means for detecting a travel state of the vehicle, image controlling means for controlling, on the basis of the detected travel state, an on-screen position to be occupied by the image produced by the image producing means, and image displaying means for displaying the image at the on-screen position controlled by the image controlling means.

The on-vehicle image display apparatus thus constructed according to the present invention can inform and remind the vehicle occupants about the current and next travel states of the vehicle by using the on-screen position of the image controlled on the basis of the travel state detected by the travel state detecting means.

In the on-vehicle image display apparatus according to the present invention, the travel state detecting means may detect an acceleration of the vehicle in at least one of longitudinal, transverse, and vertical directions of the vehicle.

The on-vehicle image display apparatus thus constructed according to the present invention can inform and remind the vehicle occupants about the current and next travel states of the vehicle by using the on-screen position of the image controlled on the basis of the acceleration detected in each of longitudinal, transverse, and vertical directions of the vehicle.

In the on-vehicle image display apparatus according to the present invention, the travel state detecting means may detect the acceleration on the basis of at least one of a velocity detected by an on-vehicle velocity sensor, an acceleration detected by an on-vehicle acceleration sensor, an angular velocity detected by an on-vehicle angular velocity sensor, and a vibration frequency detected by an on-vehicle vibration sensor.

The on-vehicle image display apparatus thus constructed according to the present invention can inform and remind the vehicle occupants about the current and next travel states of the vehicle by using the on-screen position of the image controlled on the basis of at least one of the velocity detected by the velocity sensor, the acceleration detected by the acceleration sensor, the angular velocity detected by the angular velocity sensor, and the frequency of the vibration detected by the vibration sensor.

In the on-vehicle image display apparatus according to the present invention, the travel state detecting means may detect at least one of a left turn, a right turn, an acceleration, and a deceleration of the vehicle.

The on-vehicle image display apparatus thus constructed according to the present invention can inform and remind the vehicle occupants about the current and next travel states of the vehicle by using the on-screen position of the image controlled on the basis of at least one of a left turn, a right turn, an acceleration, and a deceleration of the vehicle.

In the on-vehicle image display apparatus according to the present invention, the travel state detecting means may detect the travel state of the vehicle from an operating state of at least one of a steering wheel, an acceleration pedal, a brake pedal, a direction indicator, and a hazard indicator.

The on-vehicle image display apparatus thus constructed according to the present invention can inform and remind the vehicle occupants about the current and next travel states of the vehicle by using the on-screen position of the image controlled on the basis of an operating state of at least one of a steering wheel, an acceleration pedal, a brake pedal, an indicator, and a hazard indicator.

The on-vehicle image display apparatus according to the present invention may further comprise navigation means for producing support information to support a driver in driving to a destination through an optimum travel route. The travel state detecting means may detect the travel state of the vehicle on the basis of the support information produced by the navigation means.

The on-vehicle image display apparatus thus constructed according to the present invention can inform and remind the vehicle occupants about the current and next travel states of the vehicle by using the on-screen position of the image controlled on the basis of the support information produced by the navigation means.

In the on-vehicle image display apparatus according to the present invention, the navigation means has road map information on a road near or ahead of the vehicle. The travel state detecting means may detect the travel state of the vehicle by using the road map information.

The on-vehicle image display apparatus thus constructed according to the present invention can inform and remind the vehicle occupants about the current and next travel states of the vehicle by using the on-screen position of the image controlled on the basis of the road map information on a road near or ahead of the vehicle.

In the on-vehicle image display apparatus according to the present invention, the navigation means has horizontal direction information on a road near or ahead of the vehicle. The travel state detecting means may detect the travel state of the vehicle by using the horizontal direction information.

The on-vehicle image display apparatus thus constructed according to the present invention can inform and remind the vehicle occupants about the current and next travel states of the vehicle by using the on-screen position of the image controlled on the basis of the horizontal direction information on a road near or ahead of the vehicle.

In the on-vehicle image display apparatus according to the present invention, the navigation means has vertical direction information on a road near or ahead of the vehicle. The travel state detecting means may detect the travel state of the vehicle by using the vertical direction information.

The on-vehicle image display apparatus thus constructed according to the present invention can inform and remind the vehicle occupants about the current and next travel states of the vehicle by using the on-screen position of the image controlled on the basis of the vertical direction information on a road near or ahead of the vehicle.

The on-vehicle image display apparatus may further comprise camera means for taking, as a view from the vehicle, an image of an object near or ahead of the vehicle. The travel state detecting means may detect the travel state of the vehicle on the basis of the image taken by the camera means.

The on-vehicle image display apparatus thus constructed according to the present invention can inform and remind the vehicle occupants about the current and next travel states of the vehicle by using the on-screen position of the image controlled on the basis of the image of the object near or ahead of the vehicle.

The on-vehicle image display apparatus may further comprise two or more travel state detecting means.

The on-vehicle image display apparatus thus constructed according to the present invention can inform and remind the vehicle occupants about the current and next travel states of the vehicle by using the on-screen position of the image controlled on the basis of the travel states detected by two or more travel state detecting means.

In the on-vehicle image display apparatus according to the present invention, the travel state detecting means may detect the travel state of the vehicle by using a graded scale.

The on-vehicle image display apparatus thus constructed according to the present invention can inform and remind the vehicle occupants about the current and next travel states of the vehicle by using the on-screen position of the image controlled on the basis of the travel state of the vehicle detected on the basis of the graded scale.

In the on-vehicle image display apparatus according to the present invention, the image controlling means may shift the on-screen position in response to the travel state of the vehicle detected by the travel state detecting means.

The on-vehicle image display apparatus thus constructed according to the present invention can inform and remind the vehicle occupants about the current and next travel states of the vehicle by using the on-screen position of the image shifted in response to the travel state of the vehicle detected by the travel state detecting means.

In the on-vehicle image display apparatus according to the present invention, the image controlling means may gradually shift the on-screen position in response to the travel state detected on the basis of the graded scale by the travel state detecting means.

The on-vehicle image display apparatus thus constructed according to the present invention can inform and remind the vehicle occupants about the current and next travel states of the vehicle by using the on-screen position of the image shifted in response to the travel state detected, on the basis of the graded scale, by the travel state detecting means.

In the on-vehicle image display apparatus according to the present invention, the image controlling means may shift the on-screen position if the travel state detected on the basis of the graded scale by the travel state detecting means is equal to or larger than a predetermined threshold level.

The on-vehicle image display apparatus thus constructed according to the present invention can inform and remind the vehicle occupants about the current and next travel states of the vehicle by using the on-screen position of the image shifted in response to the travel state detected on the basis of the graded scale by the travel state detecting means if the travel state detected on the basis of the graded scale by the travel state detecting means is equal to or larger than a predetermined threshold level.

In the on-vehicle image display apparatus according to the present invention, the image controlling means may update, in response to the travel state detected on the basis of the graded scale by the travel state detecting means, a velocity at which the on-screen position of the image is shifted.

The on-vehicle image display apparatus thus constructed according to the present invention can inform and remind the vehicle occupants about the current and next travel states of the vehicle by changing, in response to the travel state detected on the basis of the graded scale by the travel state detecting means, a velocity at which the on-screen position of the image is shifted.

In the on-vehicle image display apparatus according to the present invention, the image controlling means may allow the image to be displayed at the shifted on-screen position over a predetermined period of time.

The on-vehicle image display apparatus thus constructed according to the present invention can inform and remind the vehicle occupants about the current and next travel states of the vehicle by allowing the image to be displayed at the shifted on-screen position over a predetermined period of time.

In the on-vehicle image display apparatus according to the present invention, the image controlling means may allow the image to be displayed at an original position after the image is displayed at the shifted on-screen position over the predetermined period of time.

The on-vehicle image display apparatus thus constructed according to the present invention can inform and remind the vehicle occupants about the current and next travel states of the vehicle by allowing the image to be displayed at an original position after the image is displayed at the shifted on-screen position over the predetermined period of time

In the on-vehicle image display apparatus according to the present invention, the image controlling means may shift the on-screen position at a growing pace.

The on-vehicle image display apparatus thus constructed according to the present invention can inform and remind the vehicle occupants about the current and next travel states of the vehicle by using the on-screen position of the image shifted at the growing pace.

In the on-vehicle image display apparatus according to the present invention, the image controlling means may shift the on-screen position in any one of horizontal, vertical, and oblique directions.

The on-vehicle image display apparatus thus constructed according to the present invention can inform and remind the vehicle occupants about the current and next travel states of the vehicle by using the on-screen position of the image shifted in any one of horizontal, vertical, and oblique directions.

The on-vehicle image display apparatus according to the present invention may further comprise setting means for setting whether or not to allow the image controlling means to assume an active state to control the on-screen position.

The on-vehicle image display apparatus thus constructed according to the present invention can inform and remind the vehicle occupants about the current and next travel states of the vehicle by using the on-screen position of the image controlled on the basis of the information, set by the setting means, on whether or not to allow the image controlling means to assume an active state to control the on-screen position.

The on-vehicle image display apparatus according to the present invention may further comprise setting means for setting whether or not to allow the image controlling means to assume an active state to shift the on-screen position in each of the horizontal, vertical, and oblique directions.

The on-vehicle image display apparatus thus constructed according to the present invention can inform and remind the vehicle occupants about the current and next travel states of the vehicle by using the on-screen position of the image shifted on the basis of the information, set by the setting means, on whether or not to allow the image controlling means to assume an active state to shift the on-screen position in each of the horizontal, vertical, and oblique directions.

In the on-vehicle image display apparatus according to the present invention, the setting means may set a distance between an original position and the on-screen position shifted by the image controlling means in each of the horizontal, vertical, and oblique directions.

The on-vehicle image display apparatus thus constructed according to the present invention can inform and remind the vehicle occupants about the current and next travel states of the vehicle by using the on-screen position of the image shifted on the basis of the information, set by the setting means, on a distance between an original position and the on-screen position shifted by the image controlling means in each of the horizontal, vertical, and oblique directions.

In the on-vehicle image display apparatus according to the present invention, the setting means may set a period of time over which the image controlling means shifts the on-screen position in each of the horizontal, vertical, and oblique directions.

The on-vehicle image display apparatus thus constructed according to the present invention can inform and remind the vehicle occupants about the current and next travel states of the vehicle by using the on-screen position of the image shifted on the basis of the information, set by the setting means, on a period of time over which the image controlling means shifts the on-screen position in each of the horizontal, vertical, and oblique directions.

In the on-vehicle image display apparatus according to the present invention, the setting means may set a velocity at which the image controlling means shifts the on-screen position in each of the horizontal, vertical, and oblique directions.

The on-vehicle image display apparatus thus constructed according to the present invention can inform and remind the vehicle occupants about the current and next travel states of the vehicle by using the on-screen position of the image shifted on the basis of the information, set by the setting means, on a velocity at which the image controlling means shifts the on-screen position in each of the horizontal, vertical, and oblique directions.

In the on-vehicle image display apparatus according to the present invention, the image displaying means may include a screen and an image projector for producing an image to be projected on the screen. The image controlling means may shift, in response to the travel state, an on-screen position which the image produced by the image projector is displayed on.

The on-vehicle image display apparatus thus constructed according to the present invention can inform and remind the vehicle occupants about the current and next travel states of the vehicle by using the on-screen position of the image shifted in response to the travel state.

The on-vehicle image display apparatus according to the present invention may further comprise two or more image controlling means. The setting means may set independently whether or not to allow said two or more image controlling means to assume active states to shift the on-screen position.

The on-vehicle image display apparatus thus constructed according to the present invention can inform and remind the vehicle occupants about the current and next travel states of the vehicle by using the on-screen position of the image controlled by said two or more image controlling means on the basis of information, independently set by the setting means, on whether or not to allow said two or more image controlling means to assume active states to shift the on-screen position.

### ADVANTAGEOUS EFFECT OF THE INVENTION

The on-vehicle image display apparatus according to the present invention can inform and remind the vehicle occupants about the current and next travel states of the vehicle while allowing the vehicle occupants to watch TV or the like in a moving vehicle, and reduce a burden to be imposed on the vehicle occupants by allowing the image controlling means to control the on-screen position to be occupied by the image produced by the image producing means.

### BRIEF DESCRIPTION OF THE DRAWINGS

FIG **1** is a block diagram showing the on-vehicle image display apparatus according to the first embodiment of the present invention.
FIG **2(a)** is a view schematically showing, as an example, an image to be displayed on the screen by the on-vehicle image display apparatus according to the first embodiment of the present invention while the vehicle is going straight on at a constant velocity.
FIG **2(b)** is a view schematically showing, as an example, an image to be displayed on the screen by the on-vehicle image display apparatus according to the first embodiment of the present invention while the vehicle is turning to the left.

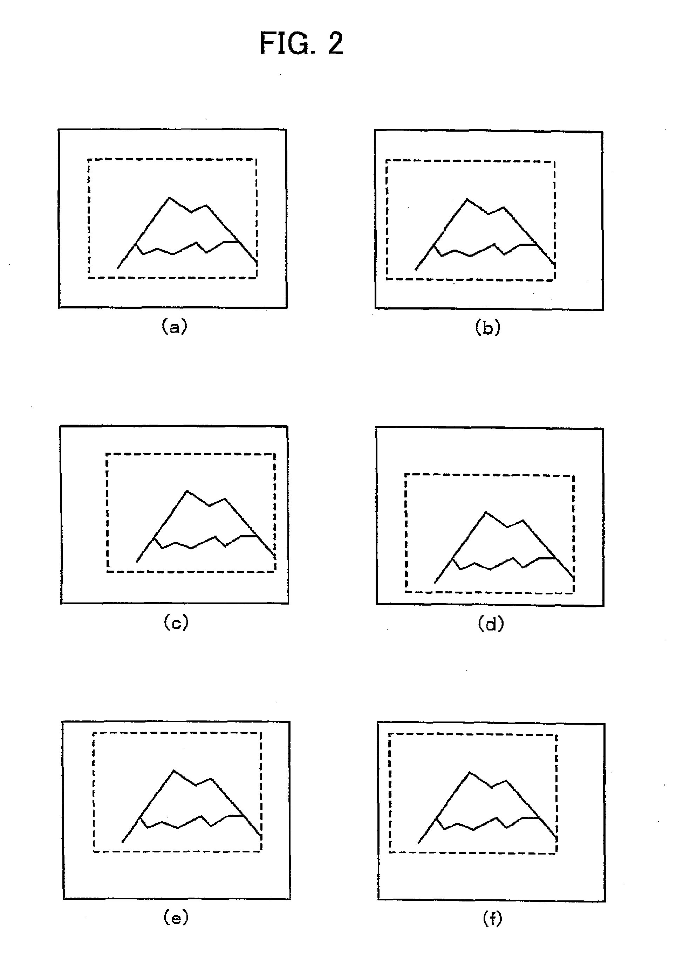

FIG 2**(c)** is a view schematically showing, as an example, an image to be displayed on the screen by the on-vehicle image display apparatus according to the first embodiment of the present invention while the vehicle is turning to the right.
FIG **2(d)** is a view schematically showing, as an example, an image to be displayed on the screen by the on-vehicle image display apparatus according to the first embodiment of the present invention while the vehicle is being decelerated.
FIG **2(e)** is a view schematically showing, as an example, an image to be displayed on the screen by the on-vehicle image display apparatus according to the first embodiment of the present invention while the vehicle is being accelerated.
FIG **2(f)** is a view schematically showing, as an example, an image to be displayed on the screen by the on-vehicle image display apparatus according to the first embodiment of the present invention while the vehicle is being rapidly accelerated while turning to the left.

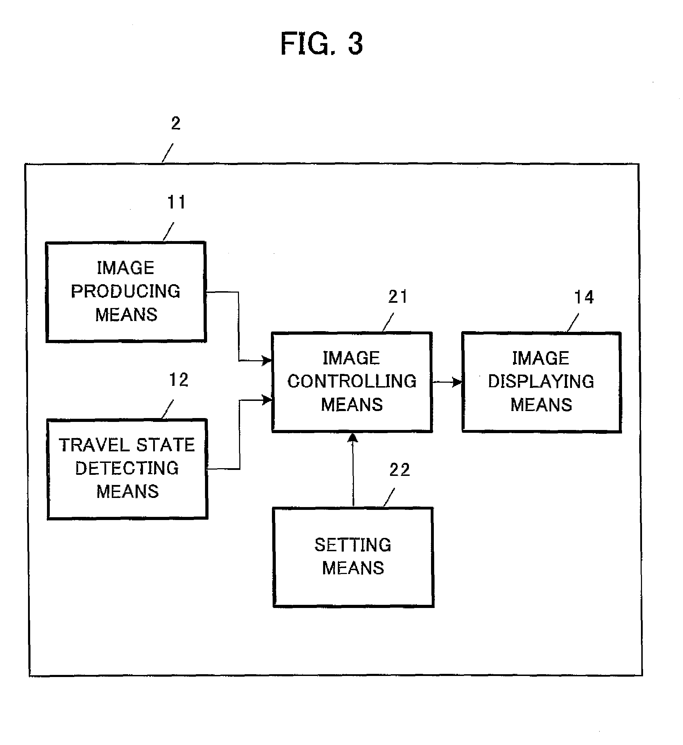

FIG **3** is a block diagram showing the on-vehicle image display apparatus according to the second embodiment of the present invention.

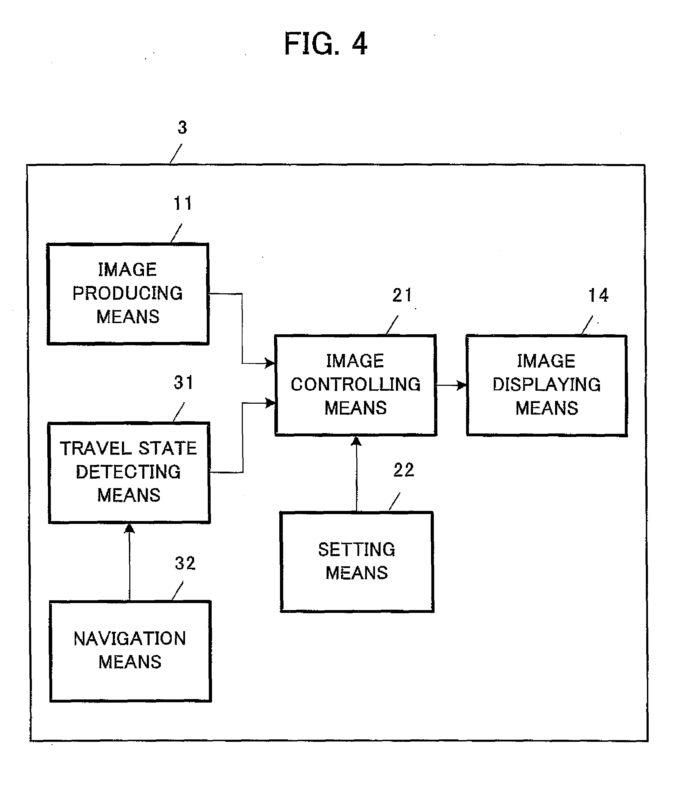

FIG **4** is a block diagram showing the on-vehicle image display apparatus according to the third embodiment of the present invention.

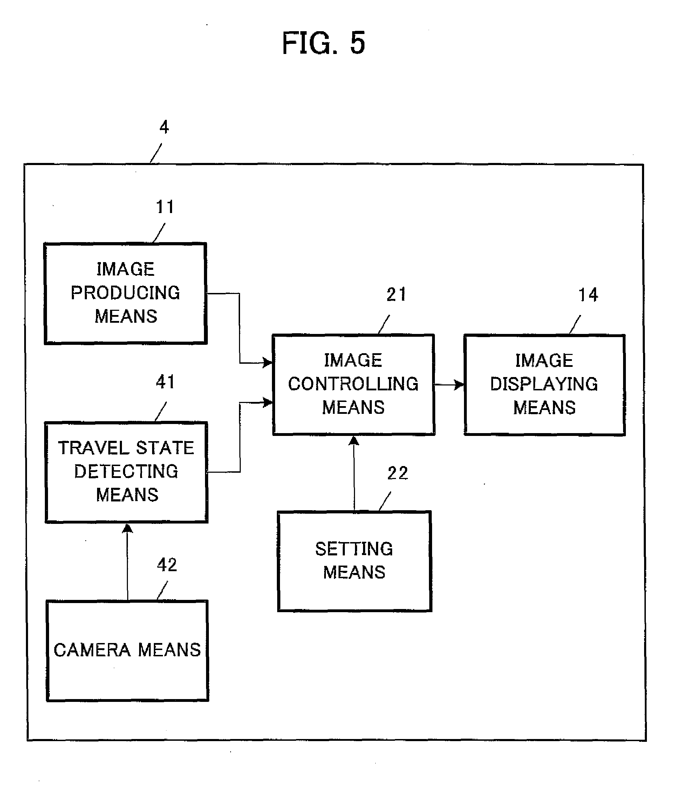

FIG. **5** is a block diagram showing the on-vehicle image display apparatus according to the fourth embodiment of the present invention.

### EXPLANATION OF THE REFERENCE NUMERALS

- **1, 2, 3,** and **4**:: on-vehicle image display apparatus
- **11:**: image producing means
- **12**:: travel state detecting means
- **13:**: image controlling means
- **14:**: image displaying means
- **21:**: image controlling means
- **22:**: setting means
- **31**:: travel state detecting means
- **32**:: navigation means
- **41:**: travel state detecting means
- **42:**: camera means

### DESCRIPTION OF THE PREFERRED EMBODIMENTS

The first to fourth embodiments of the on-vehicle image display apparatus according to the present invention will be described hereinafter with reference to the accompanying drawings.

### (First embodiment)

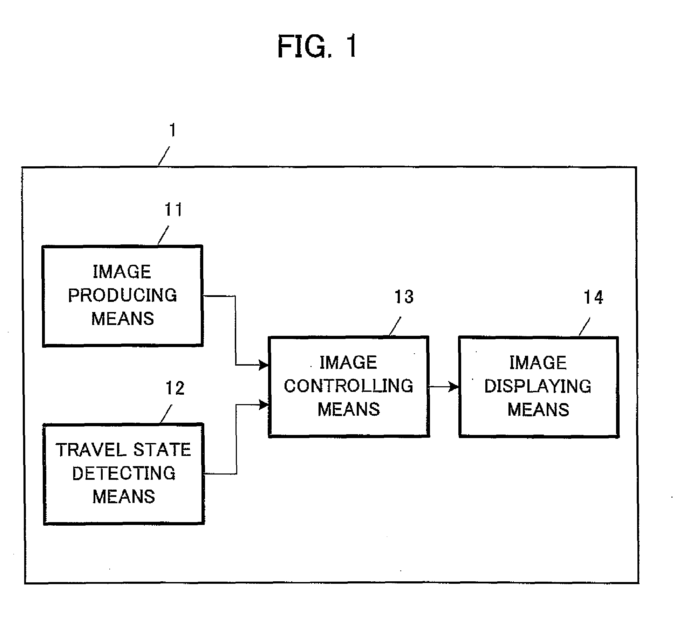

FIG. **1** is a block diagram showing the first embodiment of the on-vehicle image display apparatus according to the present invention.

As shown in FIG. **1,** the on-vehicle image display apparatus **1** comprises image producing means **11** for producing an image, image displaying means **14** for displaying, on a screen, the image produced by the image producing means **11,** travel state detecting means **12** for detecting a travel state of a vehicle, and image controlling means **13** for controlling, on the basis of the travel state detected by the travel state detecting means **12,** an on-screen position to be occupied by the image produced by the image producing means **11** if the image produced by the image producing means **11** is displayed on the screen by the image displaying means **14.**

The image producing means **11** is constituted by an apparatus such as for example a television, a video player, a DVD player, and a video game player.

The travel state detecting means **12** detects right and left turns of the vehicle on the basis of an operating state of a steering wheel or an operating state of a direction indicator, to detect a deceleration of the vehicle on the basis of an operating state of a brake pedal, to detect a stop of the vehicle on the basis of an operating state of a hazard indicator, and to detect an acceleration of the vehicle on the basis of an operating state of an acceleration pedal. Here, the travel state detecting means **12** may detect an acceleration in at least one of longitudinal, transverse, and vertical directions of the vehicle by using at least one of a velocity sensor for detecting a velocity, an acceleration sensor for detecting an acceleration, an angular velocity sensor for detecting an angular velocity, and a vibration sensor for detecting a frequency of a vibration.

If the image produced by the image producing means **11** is displayed on the screen by the image displaying means **14,** the image controlling means **13** shifts the on-screen position to be occupied by the image on the basis of the detection on whether the vehicle is turning to the right or the left, or whether the vehicle is being accelerated or decelerated, or on the basis of the acceleration detected by the travel state detecting means **12** in each of longitudinal, transverse, and vertical directions of the vehicle.

The image displaying means **14** is constituted by one or more elements such as for example a LCD (liquid crystal display) unit, a CRT (cathode ray tube) unit, an EL (electroluminescence) display unit, a plasma display unit, a projector for projecting an image on a screen, a head mounted display unit, and a head-up display unit.

The operation of the on-vehicle image display apparatus **1** will be then described hereinafter with reference to FIG. **2.**

While the vehicle is traveling in one direction at a constant velocity, the on-screen position to be occupied by the image is not shifted in any direction by the image controlling means **13** by reason that the vehicle is not being accelerated in any direction. Accordingly, the image produced by the image producing means **11** is displayed by the image displaying means **14** in the center of the screen as shown in FIG. **2(a).**

While the vehicle is turning to the left, or going around a left-hand curve, the travel state detecting means **12** detects a leftward acceleration or a condition that the driver is steering to the left. The on-screen position to be occupied by the image is shifted to the left by the image controlling means **13** on the basis of the leftward acceleration or the condition detected by the travel state detecting means **12.** The image produced by the image producing means **11** is displayed by the image displaying means **14** at the on-screen position shifted to the left as shown in FIG **2(b).**

While the vehicle is turning to the right, or going around a right-hand curve, the travel state detecting means **12** detects a rightward acceleration or a condition that the driver is steering to the right. The on-screen position to be occupied by the image is shifted to the right by the image controlling means **13** on the basis of the rightward acceleration or the condition detected by the travel state detecting means **12.** The image produced by the image producing means **11** is displayed by the image displaying means **14** at the on-screen position shifted to the right as shown in FIG. **2**(c).

While the vehicle is being accelerated in the forward direction, the travel state detecting means **12** detects a forward acceleration or a condition that the driver is accelerating the vehicle. The on-screen position to be occupied by the image is shifted to the lower side of the screen by the image controlling means **13** on the basis of the forward acceleration or the condition detected by the travel state detecting means **12.** The image produced by the image producing means **11** is displayed at the on-screen position shifted to the lower side of the screen as shown in FIG**. 2(d).**

While the vehicle is being decelerated in the forward direction, the travel state detecting means **12** detects a backward acceleration or a condition that the driver is decelerating the vehicle. The on-screen position to be occupied by the image is shifted to the upper side of the screen by the image controlling means **13** on the basis of the backward acceleration or the condition detected by the travel state detecting means **12.** The image produced by the image producing means **11** is displayed at the on-screen position shifted to the upper side of the screen as shown in FIG. **2(e).**

While the vehicle is turning to the left at a relatively high velocity, the travel state detecting means **12** detects the backward and leftward acceleration or a condition that the driver is steering to the left while driving at the relatively high velocity. The on-screen position to be occupied by the image is shifted to the upper left-hand corner of the screen by the image controlling means **13** on the basis of the backward and leftward acceleration or the condition detected by the travel state detecting means **12.** The image produced by the image producing means **11** is displayed on the on-screen position shifted to the upper left-hand corner of the screen as shown in FIG. **2(f).**

Here, the present invention does not restrict the relationship between the direction of the detected acceleration and the direction in which the on-screen position of the image is shifted by the image controlling means **13.** As shown in FIG. **2**(e), the image controlling means **13** may shift, in the upward direction of the screen, the on-screen position to be occupied by the image while the vehicle is being accelerated in the forward direction. As shown in FIG. **2(d),** the image controlling means **13** may shift, in the downward direction of the screen, the on-screen position to be occupied by the image while the vehicle is decelerated in the forward direction.

The travel state detecting means **12** may detect the travel state in a stepwise fashion, while the image controlling means **13** may shift, in the stepwise fashion, the on-screen position to be occupied by the image. If, for example, the acceleration or the velocity is rapidly increased with time, the deviation of the on-screen position to be occupied by the image may be increased by the image controlling means **13.**

The travel state detecting means **12** may detect the travel state in a stepwise fashion, while the image controlling means **13** may judge whether or not the travel state detected in the stepwise fashion exceeds a predetermined threshold level, and to shift the on-screen position to be occupied by the image if the travel state detected in the stepwise fashion exceeds a predetermined threshold level. If, on the other hand, the travel state detected in the stepwise fashion does not exceed the predetermined threshold level, the on-vehicle image display apparatus according to the first embodiment can prevent the image controlling means **13** from shifting the on-screen position to be occupied by the image.

The travel state detecting means **12** may detect the travel state in the stepwise fashion, while the image controlling means **13** to change, on the basis of the travel state detected in the stepwise fashion, a rate at which the on-screen position to be occupied by the image is shifted, and to shift, at the changed rate, the on-screen position to be occupied by the image. If, for example, the acceleration or the velocity of the vehicle exceeds a predetermined level, the image controlling means **13** may increase a rate at which the on-screen position to be occupied by the image is shifted, and to shift, at an increasing rate, the on-screen position to be occupied by the image.

The image controlling means **13** may have the image displaying means **14** display the image returned to an original position after shifting the on-screen position to be occupied by the image over a predetermined period of time.

The image controlling means **13** may return the image to the original position on the basis of the detected acceleration or the velocity after shifting the on-screen position to be occupied by the image over the period of time. For example, the image controlling means **13** may rapidly return the image to the original position, or may gradually return the image to the original position after the vehicle turns to the left or the right, or after the driver finishes accelerating or decelerating the vehicle.

Additionally, the image controlling means **13** may shift, at an accelerating rate, the on-screen position to be occupied by the image. For example, the image controlling means 13 may set a small value to the rate at the time of starting to shift the on-screen position to be occupied by the image, and may be gradually increase the rate.

From the foregoing description, it will be understood that the on-vehicle image display apparatus **1** according to the first embodiment of the present invention can inform and remind the vehicle occupants about the current and next travel states of the vehicle while allowing the vehicle occupants to watch TV or the like in a moving vehicle, and reduce a burden to be imposed on the vehicle occupants by reason that the image controlling means **13** controls, on the basis of the travel state detected by the travel state detecting means **12,** an on-screen position to be occupied by the image produced by the image producing means **11.**

Consequently, the on-vehicle image display apparatus **1** according to the first embodiment of the present invention is helpful in suppressing motion sickness. Even if a child is, without looking out of windows, focused on the screen that the image displaying means **14** is displaying the image on, the on-vehicle image display apparatus **1** according to the first embodiment of the present invention can offer advantages in motion sickness.

### (Second embodiment)

FIG. **3** is a block diagram showing the on-vehicle image display apparatus **2** according to the second embodiment of the present invention. The on-vehicle image display apparatus **2** according to the second embodiment is almost the same as the on-vehicle image display apparatus according to the first embodiment. Therefore, the constitution elements of the on-vehicle image display apparatus **2** according to the second embodiment substantially the same as those of the on-vehicle image display apparatus according to the first embodiment will not be described but bear the same reference numbers as those of the on-vehicle image display apparatus according to the first embodiment.

In this embodiment, the on-vehicle image display apparatus **2** further comprises setting means **22** for setting a method of shifting the on-screen position to be occupied by the image, a moving direction which the on-screen position of the image is shifted in, a period of time with which the on-screen position of the image is shifted, a judgment on whether or not to shift the on-screen position to be occupied by the image, and the like to the image controlling means **21.**

The setting means **22** has the image controlling means **21** selectively assume operation states including an active state to shift, in any one of the horizontal, vertical, and slanted directions of the screen, the on-screen position to be occupied by the image, and an inactive state to fix the image to a predetermined position. The following description will be firstly directed to the operation to be performed by the on-vehicle image display apparatus **2** if the image controlling means **21** is in the inactive state to shift the on-screen position to be occupied by the image in only the horizontal direction.

While the vehicle is turning to the left, or going around the left-hand curve, the leftward acceleration is detected by the travel state detecting means **12,** or the judgment is made by the travel state detecting means **12** that the driver steers to the left, the on-screen position to be occupied by the image is shifted to the left by the image controlling means **21** on the basis of the travel state detected by the travel state detecting means **12.** As shown in **FIG. 2(b),** the image is displayed on the on-screen position shifted to the left while the vehicle is turning to the left, or going around the left-hand curve.
While the vehicle is turning to the left, or going around a left-hand curve, the leftward acceleration is detected by the travel state detecting means **12,** or the judgment is made by the travel state detecting means **12** that the driver steers to the left, the on-screen position to be occupied by the image produced by the image producing means **11** is shifted to the left by the image controlling means **13** on the basis of the leftward acceleration or the travel state detected by the travel state detecting means **12.** As shown in **FIG. 2(b),** the image produced by the image producing means **11** is displayed on the on-screen position shifted to the left while the vehicle is turning to the left, or going around a left-hand curve.

While the vehicle is turning to the right, or going around the right-hand curve, the rightward acceleration is detected by the travel state detecting means **12,** or the judgment is made by the travel state detecting means **12** that the driver steers to the right, the on-screen position to be occupied by the image produced by the image producing means **11** is shifted to the right by the image controlling means **21** on the basis of the travel state detected by the travel state detecting means **12.** As shown in FIG. **2(c),** the image is displayed on the on-screen position shifted to the right while the vehicle is turning to the right, or going around the right-hand curve.

If the setting means **22** allows the image controlling means **21** to shift the on-screen position of the image only in the horizontal direction, the image controlling means **21** prevents the image from being shifted in the horizontal direction even if the vehicle is accelerated or decelerated in the forward direction.

The following description will be then directed to the case that the operation is performed by the on-vehicle image display apparatus **2** under the condition that the setting means **22** is in a state to allow the image to be shifted toward the right-hand side or the left-hand side of the screen.

While the vehicle is accelerated in the forward direction, the forward acceleration is detected by the travel state detecting means **12** or the judgment is made by the travel state detecting means **12** that the driver accelerates the vehicle in the forward direction. The on-screen position to be occupied by the image produced by the image producing means **11** is then shifted in the downward direction of the screen by the image controlling means **21** on the basis of the travel state detected by the travel state detecting means **31.** As shown in FIG. **2(d),** the image is displayed on the on-screen position sifted from its original position in the downward direction of the screen if the driver accelerates the vehicle in the forward direction.

If the vehicle is accelerated in the forward direction, the forward acceleration is detected by the travel state detecting means **12** or the judgment is made by the travel state detecting means **12** that the driver decelerates the vehicle in the forward direction. The on-screen position to be occupied by the image produced by the image producing means **11** is then shifted in the upward direction of the screen by the image controlling means **21** on the basis of the travel state detected by the travel state detecting means **31.** As shown in FIG. **2(e),** the image is displayed on the on-screen position sifted from its original position in the upward direction of the screen if the driver decelerates the vehicle in the forward direction.

If the setting means **22** is in a state to allow the image controlling means **21** to shift the on-screen position of the image only in the vertical direction, the image controlling means **21** does not shift the on-screen position of the image in the horizontal direction even if the judgment is made that the vehicle is turning to the right or the left.

Here, the present invention is not limited to the above-mentioned relationship between the direction of the detected acceleration and the direction in which the on-screen position of the image is shifted by the image controlling means **21.** As shown in FIG **2(e),** the image controlling means **21** may shift the on-screen position of the image in the upward direction of the screen if the judgment is made that the vehicle is accelerated in the forward direction. As shown in FIG. **2(d),** the image controlling means **21** may shift the on-screen position of the image in the downward direction of the screen if the judgment is made that the vehicle is decelerated in the forward direction.

The travel state detecting means **12** may detect the travel state in stages, while the setting means **22** may shift the image controlling means **21** to shift the on-screen position of the image in stages on the basis of the travel state detected in stages. The image controlling means **21** may shift the on-screen position of the image with a shift value depending on the variation of the detected acceleration or speed. The more the acceleration or the speed is rapidly changed, the more the shift value may be increased.

The travel state detecting means **12** may detect the travel state in stages, while the setting means **22** may set the image controlling means **21** to judge whether or not the travel state detected in at least one stage exceeds a predetermined threshold level, and to shift the on-screen position of the image if the judgment is made that the travel state detected in at least one stage exceeds a predetermined threshold level. If, on the other hand, the judgment is made that the travel state detected in at least one stage does not exceed a predetermined threshold level, the setting means **22** may set the image controlling means **21** not to shift the on-screen position of the image. From the foregoing description, it will be understood that the image controlling means **21** can prevent the on-screen position of the image from being varied delicately by reason that the image controlling means **21** does not shift the on-screen position of the image if the judgment is made that the acceleration or the speed of the vehicle does not exceed a predetermined threshold level.

The travel state detecting means **12** may detect the travel state in stages, while the setting means **22** may set the image controlling means **21** to change, on the basis of the travel state to be detected in each stage, a speed at which the on-screen position of the image is shifted in each stage, and to shift the on-screen position of the image with the changed speed. If, for example, the judgment is made that the acceleration or speed of the vehicle is larger than a predetermined level, the image controlling means **21** may increase a speed at which the on-screen position of the image is shifted, and to shift the on-screen position of the image with the increased speed.

The setting means **22** may set the image controlling means **22** to return the image to an original position after shifting the on-screen position of the image over the period of time.

The image controlling means **13** may set the image controlling means **22** to return the image to an original position on the basis of the detected acceleration or speed after shifting the on-screen position of the image over the period of time. The image controlling means **22** may rapidly return the image to the original position or gradually return the image to the original position after the judgment is made that the driver finishes turning the vehicle to the right turn or the left turn, accelerating, or decelerating the vehicle.

The setting means **22** may set the image controlling means **21** to shift the on-screen position of the image with an increasing speed. The image controlling means **21** may shift the on-screen position of the image with a relatively low shift value in early stage, and shift the on-screen position of the image with a shift value to be gradually increased in later stage.

In this embodiment, the on-vehicle image display apparatus further comprises a plurality of image controlling means **21.** The setting means **22** may set each of the image controlling means **21** to control the on-screen position of the image.

From the foregoing description, it will be understood that the on-vehicle image display apparatus **2** according to the second embodiment of the present invention can change, if needed, a method of controlling the on-screen position of the image by further comprising setting means for setting, to the image control means **21,** information on the control method.

### (Third embodiment)

FIG. **4** is a block diagram showing the on-vehicle image display apparatus **3** according to the third embodiment of the present invention. The on-vehicle image display apparatus **3** according to the third embodiment is almost the same as the on-vehicle image display apparatus according to the first or second embodiment. The constitution elements of the on-vehicle image display apparatus **3** according to the third embodiment substantially the same as those of the on-vehicle image display apparatus according to the first or second embodiment will not be described but bear the same reference numbers as those of the on-vehicle image display apparatus according to the first or second embodiment.

As shown in FIG. **4,** the on-vehicle image display apparatus **3** comprises navigation means **32** for producing support information to support a driver in driving to a destination through an optimum travel route on the basis of road map information and destination information. The navigation means **32** includes a memory unit having the road map information stored therein, and a current position detecting unit for detecting a current position of the vehicle by using a GPS (Global Positioning System) sensor, a gyro sensor and the like.

The navigation means **32** produces optimum travel route information by calculating an optimum travel route to a designated destination from a current position, and to assist a driver in driving to the destination through the optimum travel route by outputting support information (such as for example "please turn to the left at the next intersection" and "please turn to the right just a few meters ahead") on the basis of the optimum travel route information at the right time. The travel state detecting means **31** judges, on the basis of the support information produced by the navigation means **32,** whether or not the vehicle is about to turn to the left or the right in the road ahead of the vehicle, and to output information on this judgment to the image controlling means **21.** The image controlling means **21** shifts, on the basis of the information received from the travel state detecting means **31,** the on-screen position of the image. From the foregoing description, it will be understood that the on-vehicle image display apparatus can shift the on-screen position of the image before the vehicle turns to the left or the right in the road ahead.

If, for example, the travel state detecting means **31** makes, on the basis of the support information produced by the navigation means **32,** a judgment that the vehicle is about to turn to the left, or to go around a left-hand curve in the road ahead, the on-screen position to be occupied by the image is shifted to the right. As shown in FIG **2(b),** the image produced by the image producing means **11** is displayed by the image displaying means **14** at the on-screen position shifted to the left.

If travel state detecting means **31** makes, on the basis of the support information produced by the navigation means **32,** a judgment that the vehicle turns to the right, or goes around a right-hand curve in the road ahead, the on-screen position to be occupied by the image is shifted to the right. As shown in **FIG 2(c),** the image produced by the image producing means **11** is displayed by the image displaying means **14** at the on-screen position shifted to the right.

Additionally, the setting means **22** may set the image controlling means **21** to start to shift the on-screen position of the image if the judgment is made, on the basis of the travel route information received from the navigation means **32,** that the vehicle comes to a designated point spaced apart from an intersection where the vehicle is about to turn to the right or the left.

The navigation means **32** may output road map information on altitude (i.e. whether or not the vehicle is about to drive along an upward-sloping road, or a downward-sloping road). The travel state detecting means **31** may detect, on the basis of the road map information received from the navigation means **32,** whether or not the vehicle is about to be accelerated in a downward-sloping road ahead, or decelerated in an upward-sloping road ahead.

If the judgment is made by the travel state detecting means **31** that the vehicle is accelerated on the basis of the travel route information produced by the navigation means **32,** the on-screen position to be occupied by the image produced by the image producing means **11** is shifted in the downward direction of the screen by the image controlling means **21** on the basis of the travel state detected by the travel state detecting means **31.** As shown in FIG **2(d)**, the image displayed on the screen by the image displaying means **14** is out of the original position in the downward direction of the screen if the vehicle is accelerated.

If the judgment is made by the travel state detecting means **31** that the vehicle is decelerated on the basis of the travel route information produced by the navigation means **32,** the on-screen position to be occupied by the image produced by the image producing means **11** is shifted in the upward direction of the screen by the image controlling means **21** on the basis of the travel state detected by the travel state detecting means **31.** As shown in FIG. **2(e),** the image displayed on the screen by the image displaying means **14** is out of the original position in the upward direction of the screen if the vehicle is decelerated.

Here, the present invention is not limited to the above-mentioned relationship between the direction of the detected acceleration and the direction in which the on-screen position of the image is shifted by the image controlling means **21.** As shown in FIG. 2(e), the image controlling means **21** may shift the on-screen position of the image in the upward direction of the screen of the image displaying means **14** if the judgment is made that the vehicle is accelerated in the forward direction. As shown in FIG **2(d)**, the image controlling means **21** may shift the on-screen position of the image in the downward direction of the screen of the image displaying means **14** if the judgment is made that the vehicle is decelerated in the forward direction.

If the navigation means **32** is in an inactive state not to support a driver in driving to a destination through an optimum travel route, the travel state detecting means **31** may receive road map information from the navigation means **12,** and to detect features of the road near or ahead of the vehicle on the basis of on the basis of the received road map information, and information on its current position and traveling direction. The image controlling means **21** may have the image displaying means **14** shift the on-screen position of the image by detecting right-hand and left-hand curves, or changes in altitude (i.e. whether or not the vehicle is about to drive along an upward-sloping road, or a downward-sloping road).

The travel state detecting means **12** may detect the travel state in stages, while the setting means **22** may shift the image controlling means **21** to shift the on-screen position of the image in stages on the basis of the travel state detected in stages. The image controlling means **21** may shift the on-screen position of the image with a shift value depending on the variation of the detected acceleration or speed. The more the acceleration or the speed is rapidly changed, the more the shift value may be increased.

The travel state detecting means **12** may detect the travel state in stages, while the setting means **22** may set the image controlling means **21** to judge whether or not the travel state detected in at least one stage exceeds a predetermined threshold level, and to shift the on-screen position of the image if the judgment is made that the travel state detected in at least one stage exceeds a predetermined threshold level. If, on the other hand, the judgment is made that the travel state detected in at least one stage does not exceed a predetermined threshold level, the setting means **22** may set the image controlling means **21** not to shift the on-screen position of the image. From the foregoing description, it will be understood that the image controlling means **21** can prevent the on-screen position of the image from being varied delicately by reason that the image controlling means 21 does not shift the on-screen position of the image if the judgment is made that the acceleration or the speed of the vehicle does not exceed a predetermined threshold level.

The travel state detecting means **12** may detect the travel state in stages, while the setting means **22** may set the image controlling means **21** to change, on the basis of the travel state to be detected in each stage, a speed at which the on-screen position of the image is shifted in each stage, and to shift the on-screen position of the image with the changed speed. If, for example, the judgment is made that the acceleration or speed of the vehicle is larger than a predetermined level, the image controlling means **21** may increase a speed at which the on-screen position of the image is shifted, and to shift the on-screen position of the image with the increased speed.

The setting means **22** may set the image controlling means **22** to return the image to an original position after shifting the on-screen position of the image over the period of time.

The image controlling means **13** may set the image controlling means **22** to return the image to an original position on the basis of the detected acceleration or speed after shifting the on-screen position of the image over the period of time. The image controlling means **22** may rapidly return the image to the original position or gradually return the image to the original position after the judgment is made that the driver finishes turning the vehicle to the right turn or the left turn, accelerating, or decelerating the vehicle.

The setting means **22** may set the image controlling means **21** to shift the on-screen position of the image with an increasing speed. The image controlling means **21** may shift the on-screen position of the image with a relatively low shift value in early stage, and shift the on-screen position of the image with a shift value to be gradually increased in later stage.

In this embodiment, the on-vehicle image display apparatus further comprise a plurality of image controlling means **21.** The setting means **22** may set each of the image controlling means **21** to control the on-screen position of the image.

From the foregoing description, it will be understood that the on-vehicle image display apparatus 3 according to the third embodiment of the present invention can shift the on-screen position of the image, if the vehicle is about to turn to the right or the left, or about to be accelerated or decelerated, by reason that the travel state detecting means 31 detects, on the basis of the travel route information produced by the navigation means 32, whether or not the vehicle is about to turn to the right or the left, or whether the vehicle is about to be accelerated or decelerated, before the vehicle starts to turn to the right or the left, or starts to be accelerated or decelerated.

### (Fourth embodiment)

**FIG. 5** is a block diagram showing the on-vehicle image display apparatus **4** according to the fourth embodiment of the present invention. The on-vehicle image display apparatus **4** according to the fourth embodiment is almost the same as the on-vehicle image display apparatus according to the first or second embodiment. Therefore, the constitution elements of the on-vehicle image display apparatus 4 according to the fourth embodiment substantially the same as those of the on-vehicle image display apparatus according to the first or second embodiment will not be described but bear the same reference numbers as those of the on-vehicle image display apparatus according to the first or second embodiment.

In this embodiment, the on-vehicle image display apparatus 4 further comprises, as one of features, camera means **42** pointed at an object ahead of a vehicle.

The camera means **42** takes an image indicative of the object ahead of the vehicle, and to output the image to the travel state detecting means **41.** The travel state detecting means **41** detects a center line, a white line dividing a sidewalk from a vehicular road, and the like from the image taken by the camera means **42,** and to detect, on the basis of the image taken by the camera means **42,** whether the vehicle turns to the left, or turns to the right in the road ahead, and whether the vehicle goes up a slope, or goes down a slope in the road ahead. From the foregoing description, it will be understood that the image controlling means **21** can shift the on-screen position to be occupied by the image (produced by the image producing means 11), if the vehicle turns to the left, or turns to the right in the road ahead, by reason that the travel state detecting means **41** detects, on the basis of the image taken by the camera means **42,** whether the vehicle turns to the left, or turns to the right in the road ahead.

If, for example, the travel state detecting means **41** makes, on the basis of the image taken by the camera means **42,** an estimation that the vehicle turns to the left, or goes around a left-hand curve in the road ahead, the on-screen position to be occupied by the image (produced by the image producing means **11)** is shifted to the left on the basis of the travel state detected by the travel state detecting means **12.** The image produced by the image producing means **11** is then displayed at the on-screen position shifted to the left. As shown in FIG **2(b),** the image somewhat deviated from an original position is displayed on the screen by the image display means **14** if the vehicle is about to turn to the left, or to go around the left-hand curve.

If, on the other hand, the judgment is made, on the basis of the image taken by the camera means **42,** by the travel state detecting means **41** that the vehicle turns to the right, or goes around a right-hand curve in the road ahead, the on-screen position to be occupied by the image (produced by the image producing means **11)** is shifted to the right on the basis of the travel state detected by the travel state detecting means **12.** The image produced by the image producing means 11 is then displayed at the on-screen position shifted to the right. As shown in **FIG. 2(c),** the image somewhat deviated from the original position is displayed on the screen by the image display means 14 if the vehicle is about to turn to the right or to go around the right-hand curve.

In order to have the image controlling means **21** shift the on-screen position of the image at the right time on the basis of the image taken by the camera means **42,** the on-vehicle image display apparatus **4** may further comprise setting means **22** for setting a distance between a position at which the image controlling means **21** starts to shift the on-screen position to be occupied by the image (produced by the image producing means **11),** and a position at which the vehicle starts to turn to the right, or to turn to the left.

The travel state detecting means **41** can judge whether the vehicle is accelerated, or decelerated in the road ahead, by judging, on the basis of the image taken by the camera means **42,** whether or not there is an upward slope in the road ahead (it appears that the road is extended in an upward direction on the image), or whether or not there is a downward slope in the road ahead (it appears that the road disappears on the image). If, for example, the upward slope is detected from the image taken by the camera means **42,** the travel state detecting means **41** makes a judgment that the vehicle is decelerated in the road ahead. If, on the other hand, the downward slope is detected from the image taken by the camera means **42,** the travel state detecting means **41** makes a judgment that the vehicle is accelerated in the road ahead.

If the travel state detecting means **41** makes, on the basis of the image taken by the camera means **42,** a judgment that the vehicle is accelerated in the road ahead, the on-screen position to be occupied by the image (produced by the image producing means **11)** is shifted in a downward direction by the image controlling means **21.** The image produced by the image producing means **11** is then displayed by the image displaying means **14** at the on-screen position shifted in the downward direction. As shown in FIG **2(d)**, the image downwardly deviated from the original position is displayed on the screen by the image display means 14 if the travel state detecting means **41** makes a judgment that the vehicle is accelerated in the road ahead.

If, on the other hand, the travel state detecting means **41** makes, on the basis of the image taken by the camera means **42,** a judgment that the vehicle is decelerated in the road ahead, the on-screen position to be occupied by the image (produced by the image producing means **11)** is shifted in an upward direction by the image controlling means **21.** The image produced by the image producing means **11** is then displayed by the image displaying means **14** at the on-screen position shifted in the upward direction. As shown in FIG. **2(e),** the image upwardly deviated from the original position is displayed on the screen by the image display means **14** if the travel state detecting means **41** makes a judgment that the vehicle is decelerated in the road ahead.

Here, the present invention does not restrict the relationship between a direction (in which the vehicle is accelerated in the road ahead) and a direction (in which the on-screen position to be occupied by the image is shifted by the image controlling means **21).** As shown in FIG. **2(e),** the image controlling means **21** may shift, in the upward direction, the on-screen position to be occupied by the image if the travel state detecting means **41** makes a judgment that the vehicle is accelerated in the road ahead. As shown in FIG. **2(d),** the image controlling means **21** may shift, in the downward direction, the on-screen position to be occupied by the image if the travel state detecting means **41** makes a judgment that the vehicle is decelerated in the road ahead.

In this embodiment, the travel state detecting means **12** detects the white line from the image taken by the camera means **42**. However, the travel state detecting means **12** may detect, on the basis of a movement of a vehicle in the road ahead, whether the vehicle turns to the right, or to the left in the road ahead, or whether the vehicle is accelerated, or decelerated in the road ahead.

The travel state detecting means **12** may detect the travel state in a stepwise fashion, while the setting means **22** may shift the image controlling means **21** to shift the on-screen position of the image in stages on the basis of the travel state detected in stages. The image controlling means **21** may shift the on-screen position of the image with a shift value depending on the variation of the detected acceleration or speed. The more the acceleration or the speed is rapidly changed, the more the shift value may be increased.

The travel state detecting means **12** may detect the travel state in stages, while the setting means **22** may set the image controlling means **21** to judge whether or not the travel state detected in at least one stage exceeds a predetermined threshold level, and to shift the on-screen position of the image if the judgment is made that the travel state detected in at least one stage exceeds a predetermined threshold level. If, on the other hand, the judgment is made that the travel state detected in at least one stage does not exceed a predetermined threshold level, the setting means **22** may set the image controlling means **21** not to shift the on-screen position of the image. From the foregoing description, it will be understood that the image controlling means **21** can prevent the on-screen position of the image from being varied delicately by reason that the image controlling means 21 does not shift the on-screen position of the image if the judgment is made that the acceleration or the speed of the vehicle does not exceed a predetermined threshold level.

The travel state detecting means **12** may detect the travel state in stages, while the setting means **22** may set the image controlling means **21** to change, on the basis of the travel state to be detected in each stage, a speed at which the on-screen position of the image is shifted in each stage, and to shift the on-screen position of the image with the changed speed. If, for example, the judgment is made that the acceleration or speed of the vehicle is larger than a predetermined level, the image controlling means **21** may increase a speed at which the on-screen position of the image is shifted, and to shift the on-screen position of the image with the increased speed.

The setting means **22** may set the image controlling means **23** to return the image to an original position after shifting the on-screen position of the image over the period of time.

The setting means **22** may set the image controlling means **23** to return the image to an original position on the basis of the detected acceleration or speed after shifting the on-screen position of the image over the period of time. The image controlling means **23** may rapidly return the image to the original position or gradually return the image to the original position after the judgment is made that the driver finishes turning the vehicle to the right turn or the left turn, accelerating, or decelerating the vehicle.

The setting means **22** may set the image controlling means **21** to shift the on-screen position of the image with an increasing speed. The image controlling means **21** may shift the on-screen position of the image with a relatively low shift value in early stage, and shift the on-screen position of the image with a shift value to be gradually increased in later stage.

In this embodiment, the on-vehicle image display apparatus further comprise a plurality of image controlling means **21.** The setting means **22** may set each of the image controlling means **21** to control the on-screen position of the image.

From the foregoing description, it will be understood that the on-vehicle image display apparatus **4** according to the fourth embodiment of the present invention can shift the on-screen position of the image, if the vehicle is about to turn to the right or the left, or about to be accelerated or decelerated, by reason that the travel state detecting means **41** detects, on the basis of the image taken by the camera means **42,** whether or not the vehicle is about to turn to the right or the left, or whether the vehicle is about to be accelerated or decelerated, before the vehicle starts to turn to the right or the left, or starts to be accelerated or decelerated.

### INDUSTRIAL APPLICABILITY OF THE PRESENT INVENTION

As will be seen from the foregoing description, the on-vehicle image display apparatus according to the present invention has an advantageous effect of informing and reminding the vehicle occupants about the current and next travel states of the vehicle while allowing the vehicle occupants to watch TV or the like in a moving vehicle, and reduce a burden to be imposed on the vehicle occupants. The present invention is available as an image display apparatus to be mounted on a vehicle, and for displaying on a screen an image to be watched by vehicle occupants.

## Claims

1. An on-vehicle image display apparatus to be mounted on a vehicle, comprising:
image producing means for producing an image;
travel state detecting means for detecting a travel state of said vehicle;
image controlling means for controlling, on the basis of said detected travel state, an on-screen position to be occupied by said image produced by said image producing means; and
image displaying means for displaying said image at said on-screen position controlled by said image controlling means.

2. The on-vehicle image display apparatus as set forth in claim 1, in which said travel state detecting means is adapted to detect an acceleration of said vehicle in at least one of longitudinal, transverse, and vertical directions of said vehicle.

3. The on-vehicle image display apparatus as set forth in claim 2, in which said travel state detecting means is adapted to detect said acceleration on the basis of at least one of a velocity detected by an on-vehicle velocity sensor, an acceleration detected by an on-vehicle acceleration sensor, an angular velocity detected by an on-vehicle angular velocity sensor, and a vibration frequency detected by an on-vehicle vibration sensor.

4. The on-vehicle image display apparatus as set forth in claim 1, in which said travel state detecting means is adapted to detect at least one of a left turn, a right turn, an acceleration, and a deceleration of said vehicle.

5. The on-vehicle image display apparatus as set forth in claim 4, in which said travel state detecting means is adapted to detect said travel state of said vehicle from an operating state of at least one of a steering wheel, an acceleration pedal, a brake pedal, a direction indicator, and a hazard indicator.

6. The on-vehicle image display apparatus as set forth in claim 1, which further comprises navigation means for producing support information to support a driver in driving to a destination through an optimum travel route, and in which
said travel state detecting means is adapted to detect said travel state of said vehicle on the basis of said support information produced by said navigation means.

7. The on-vehicle image display apparatus as set forth in claim 6, in which said navigation means has road map information on a road near or ahead of said vehicle, and in which
said travel state detecting means is adapted to detect said travel state of said vehicle by using said road map information.

8. The on-vehicle image display apparatus as set forth in claim 6, in which said navigation means has horizontal direction information on a road near or ahead of said vehicle, and in which
said travel state detecting means is adapted to detect said travel state of said vehicle by using said horizontal direction information.

9. The on-vehicle image display apparatus as set forth in claim 6, in which said navigation means has vertical direction information on a road near or ahead of said vehicle, and in which
said travel state detecting means is adapted to detect said travel state of said vehicle by using said vertical direction information.

10. The on-vehicle image display apparatus as set forth in claim 1, which further comprises camera means for taking, as a view from said vehicle, an image of an object near or ahead of said vehicle, and in which
said travel state detecting means is adapted to detect said travel state of said vehicle on the basis of said image taken by said camera means.

11. The on-vehicle image display apparatus as set forth in any one of claims 1 to 10, which further comprises two or more travel state detecting means.

12. The on-vehicle image display apparatus as set forth in any one of claims 1 to 10, in which said travel state detecting means is adapted to detect said travel state of the vehicle by using a graded scale.

13. The on-vehicle image display apparatus as set forth in any one of claims 1 to 10, in which said image controlling means is adapted to shift said on-screen position in response to said travel state detected by said travel state detecting means.

14. The on-vehicle image display apparatus as set forth in any one of claim 12, in which said image controlling means is adapted to shift, in a stepwise fashion, said on-screen position in response to said travel state detected on the basis of said graded scale by said travel state detecting means.

15. The on-vehicle image display apparatus as set forth in claim 12, in which said image controlling means is adapted to shift said on-screen position if said travel state detected on the basis of said graded scale by said travel state detecting means is equal to or larger than a predetermined threshold level.

16. The on-vehicle image display apparatus as set forth in claim 12, in which said image controlling means is adapted to update, in response to said travel state detected on the basis of said graded scale by said travel state detecting means, a velocity at which said on-screen position is shifted.

17. The on-vehicle image display apparatus as set forth in claim 13, in which said image controlling means is adapted to allow said image to be displayed at said shifted on-screen position over a predetermined period of time.

18. The on-vehicle image display apparatus as set forth in claim 13, in which said image controlling means is adapted to allow said image to be displayed at an original position after said image is displayed at said shifted on-screen position over said predetermined period of time.

19. The on-vehicle image display apparatus as set forth in claim 13, in which said image controlling means is adapted to shift said on-screen position at a growing pace.

20. The on-vehicle image display apparatus as set forth in claim 13, in which said image controlling means is adapted to shift said on-screen position in any one of horizontal, vertical, and oblique directions.

21. The on-vehicle image display apparatus as set forth in claims 1 to 10, which further comprises setting means for setting whether or not to allow said image controlling means to assume an active state to control said on-screen position.

22. The on-vehicle image display apparatus as set forth in claim 20, which further comprises setting means for setting whether or not to allow said image controlling means to assume an active state to shift said on-screen position in each of said horizontal, vertical, and oblique directions.

23. The on-vehicle image display apparatus as set forth in claim 22, in which said setting means is adapted to set a distance between an original position and said on-screen position shifted by said image controlling means in each of said horizontal, vertical, and oblique directions.

24. The on-vehicle image display apparatus as set forth in claim 22, in which said setting means is adapted to set a period of time over which said image controlling means shifts said on-screen position in each of said horizontal, vertical, and oblique directions.

25. The on-vehicle image display apparatus as set forth in claim 22, in which said setting means is adapted to set a velocity at which said image controlling means shifts said on-screen position in each of said horizontal, vertical, and oblique directions.

26. The on-vehicle image display apparatus as set forth in any one of claims 1 to 10, in which said image displaying means includes a screen and an image projector for producing an image to be projected on said screen, and in which
said image controlling means is adapted to shift, in response to said travel state, an on-screen position which said image produced by said image projector is displayed on.

27. The on-vehicle image display apparatus as set forth in claim 1, which further comprises two or more image controlling means,
said setting means is adapted to set independently whether or not to allow said two or more image controlling means to assume active states to shift said on-screen position.

## Amended claims

### Amended claims under Art. 19.1 PCT

**1.** original): An on-vehicle image display apparatus to be mounted on a vehicle, comprising:
image producing means for producing an image;
travel state detecting means for detecting a travel state of said vehicle;
image controlling means for controlling, on the basis of said detected travel state, an on-screen position to be occupied by said image produced by said image producing means; and
image displaying means for displaying said image at said on-screen position controlled by said image controlling means.

**2.** original): The on-vehicle image display apparatus as set forth in claim 1, in which said travel state detecting means is adapted to detect an acceleration of said vehicle in at least one of longitudinal, transverse, and vertical directions of said vehicle.

**3.** original): The on-vehicle image display apparatus as set forth in claim 2, in which said travel state detecting means is adapted to detect said acceleration on the basis of at least one of a velocity detected by an on-vehicle velocity sensor, an acceleration detected by an on-vehicle acceleration sensor, an angular velocity detected by an on-vehicle angular velocity sensor, and a vibration frequency detected by an on-vehicle vibration sensor.

**4.** original): The on-vehicle image display apparatus as set forth in claim 1, in which said travel state detecting means is adapted to detect at least one of a left turn, a right turn, an acceleration, and a deceleration of said vehicle.

**5.** original): The on-vehicle image display apparatus as set forth in claim 4, in which said travel state detecting means is adapted to detect said travel state of said vehicle from an operating state of at least one of a steering wheel, an acceleration pedal, a brake pedal, a direction indicator, and a hazard indicator.

**6.** original): The on-vehicle image display apparatus as set forth in claim 1, which further comprises navigation means for producing support information to support a driver in driving to a destination through an optimum travel route, and in which
said travel state detecting means is adapted to detect said travel state of said vehicle on the basis of said support information produced by said navigation means.

**7.** original): The on-vehicle image display apparatus as set forth in claim 6, in which said navigation means has road map information on a road near or ahead of said vehicle, and in which
said travel state detecting means is adapted to detect said travel state of said vehicle by using said road map information.

**8.** original): The on-vehicle image display apparatus as set forth in claim 6, in which said navigation means has horizontal direction information on a road near or ahead of said vehicle, and in which
said travel state detecting means is adapted to detect said travel state of said vehicle by using said horizontal direction information.

**9.** original): The on-vehicle image display apparatus as set forth in claim 6, in which said navigation means has vertical direction information on a road near or ahead of said vehicle, and in which
said travel state detecting means is adapted to detect said travel state of said vehicle by using said vertical direction information.

**10.** original): The on-vehicle image display apparatus as set forth in claim 1, which further comprises camera means for taking, as a view from said vehicle, an image of an object near or ahead of said vehicle, and in which
said travel state detecting means is adapted to detect said travel state of said vehicle on the basis of said image taken by said camera means.

**11.** currently amended): The on-vehicle image display apparatus as set forth in claim 1, which further comprises two or more travel state detecting means.

**12.** currently amended): The on-vehicle image display apparatus as set forth in claim 1, in which said travel state detecting means is adapted to detect said travel state of the vehicle by using a graded scale.

**13.** currently amended): The on-vehicle image display apparatus as set forth in claim 1, in which said image controlling means is adapted to shift said on-screen position in response to said travel state detected by said travel state detecting means.

**14.** original): The on-vehicle image display apparatus as set forth in any one of claim 12, in which said image controlling means is adapted to shift, in a stepwise fashion, said on-screen position in response to said travel state detected on the basis of said graded scale by said travel state detecting means.

**15.** original): The on-vehicle image display apparatus as set forth in claim **12,** in which said image controlling means is adapted to shift said on-screen position if said travel state detected on the basis of said graded scale by said travel state detecting means is equal to or larger than a predetermined threshold level.

**16.** original): The on-vehicle image display apparatus as set forth in claim 12, in which said image controlling means is adapted to update, in response to said travel state detected on the basis of said graded scale by said travel state detecting means, a velocity at which said on-screen position is shifted.

**17.** original): The on-vehicle image display apparatus as set forth in claim 13, in which said image controlling means is adapted to allow said image to be displayed at said shifted on-screen position over a predetermined period of time.

**18.** original): The on-vehicle image display apparatus as set forth in claim 13, in which said image controlling means is adapted to allow said image to be displayed at an original position after said image is displayed at said shifted on-screen position over said predetermined period of time.

**19.** original): The on-vehicle image display apparatus as set forth in claim 13, in which said image controlling means is adapted to shift said on-screen position at a growing pace.

**20.** original): The on-vehicle image display apparatus as set forth in claim 13, in which said image controlling means is adapted to shift said on-screen position in any one of horizontal, vertical, and oblique directions.

**21.** currently amended): The on-vehicle image display apparatus as set forth in claim 1, which further comprises setting means for setting whether or not to allow said image controlling means to assume an active state to control said on-screen position.

**22.** currently amended): The on-vehicle image display apparatus as set forth in claim 1, which further comprises setting means for setting whether or not to allow said image controlling means to assume an active state to shift said on-screen position in each of said horizontal, vertical, and oblique directions.

**23.** currently amended): The on-vehicle image display apparatus as set forth in claim 1, in which said setting means is adapted to set a distance between an original position and said on-screen position shifted by said image controlling means in each of said horizontal, vertical, and oblique directions.

**24.** currently amended): The on-vehicle image display apparatus as set forth in claim 1, in which said setting means is adapted to set a period of time over which said image controlling means shifts said on-screen position in each of said horizontal, vertical, and oblique directions.

**25.** currently amended): The on-vehicle image display apparatus as set forth in claim 1, in which said setting means is adapted to set a velocity at which said image controlling means shifts said on-screen position in each of said horizontal, vertical, and oblique directions.

**26.** currently amended): The on-vehicle image display apparatus as set forth in claim 1, in which said image displaying means includes a screen and an image projector for producing an image to be projected on said screen, and in which
said image controlling means is adapted to shift, in response to said travel state, an on-screen position which said image produced by said image projector is displayed on.

**27.** currently amended): The on-vehicle image display apparatus as set forth in claim 21, which further comprises two or more image controlling means,
said setting means is adapted to set independently whether or not to allow said two or more image controlling means to assume active states to shift said on-screen position.
